# EUROPEAN PATENT SPECIFICATION

(11) **EP 3 541 951 B1**
(45) Date of publication and mention of the grant of the patent: **20.11.2024**
(21) Application number: 17872140.3
(22) Date of filing: 14.11.2017
(51) Int. Cl.: C40B 40/06, C40B 50/06, C12Q 1/6827, C12Q 1/6874, C12Q 1/6886, C12N 15/10

(54) **NON-UNIQUE BARCODES IN A GENOTYPING ASSAY**
NICHT EINDEUTIGE STRICHCODES IN EINEM GENOTYPISIERUNGSTEST
CODES-BARRES NON UNIQUES DANS UN TEST DE GÉNOTYPAGE

(30) Priority: 15.11.2016 US 201662422355 P
(43) Date of publication of application: 25.09.2019
(73) Proprietor: Personal Genome Diagnostics Inc., Baltimore, MD 21224 (US)
(72) Inventor: SAUSEN, Mark, Baltimore, MD 21224 (US); VELCULESCU, Victor, Baltimore, MD 21036 (US); DIAZ, Luis, Ellicott City, MD 21043 (US)
(74) Representative: Kilburn & Strode LLP
(86) International application number: PCT/US2017/061447
(87) International publication number: WO 2018/093744

(56) References cited:
- US-A1- 2014 065 621
- US-A1- 2014 066 317
- US-A1- 2016 273 049
- AARON M NEWMAN ET AL: "Integrated digital error suppression for improved detection of circulating tumor DNA", NATURE BIOTECHNOLOGY, vol. 34, no. 5, 28 March 2016 (2016-03-28), us, pages 547 - 555, XP055464348, ISSN: 1087-0156, DOI: 10.1038/nbt.3520
- I. KINDE ET AL: "Detection and quantification of rare mutations with massively parallel sequencing", PROCEEDINGS OF THE NATIONAL ACADEMY OF SCIENCES, vol. 108, no. 23, 17 May 2011 (2011-05-17), pages 9530 - 9535, XP055164202, ISSN: 0027-8424, DOI: 10.1073/pnas.1105422108
- KINDE, ISAAC ET AL.: "Detection and quantification of rare mutations with massively parallel sequencing", PROCEEDINGS OF THE NATIONAL ACADEMY OF SCIENCE, U.S.A., vol. 108, no. 23, 7 June 2011 (2011-06-07), pages 9530 - 9535, XP055164202
- PENG, QUAN ET AL.: "Reducing amplification artifacts in high multiplex amplicon sequencing by using molecular barcodes", BMC GENOMICS, vol. 16, 7 August 2015 (2015-08-07), pages 1 - 12, XP021224943
- NEWMAN, ARON M ET AL.: "Integrated digital error suppression for improved detection of circulating tumor DNA", NATURE BIOTECHNOLOGY, vol. 34, no. 5, May 2016 (2016-05-01), pages 547 - 555, XP055464348

## Description

### Field of the Invention

The invention generally involves barcoding strategies for analyzing nucleic acids for tumor-specific biomarkers.

### Background

Cancer causes more than a half a million deaths each year in the United States alone. The success of current treatments depends on the type of cancer and the stage at which it is detected. Many treatments include costly and painful surgeries and chemotherapies, and are often unsuccessful.

Early and accurate detection of mutations is essential for effective cancer therapy. One promising area in personalized cancer therapy is the analysis of circulating tumor DNA (ctDNA). ctDNA is released from tumor tissue into the blood, carries tumor specific genetic alterations, and can be analyzed through noninvasive liquid biopsy approaches to identify genetic alterations in cancer patients. Liquid biopsies offer a considerable advantage as they may eliminate the need for invasive procedures, allow early measurement of therapeutic response, and allow detection of alterations in multiple metastatic lesions over the course of therapy.

However, interrogating ctDNA in the blood has been problematic due to current limitations in genotyping technology. The fraction of ctDNA obtained from a blood sample is often very low (<1.0%) and can be difficult to detect. Most methods for evaludating ctDNA interrogate single hot spot mutations or only a few genetic alterations. Conventional genotyping in cell-free DNA has an error rate of about 1%, which makes it difficult or impossible to identify mutations with <1% prevalence in the sample using conventional molecular barcoding techniques. Current methods do not provide sufficient analytical sensitivity and specificity.

Newman et al., "Integrated digital error suppression for improved detection of circulating tumor DNA", Nat. Bio., 2016; 34(5) describes biopsy-free tumor genotyping and disease monitoring of non-small cell lung cancer patients (NSCLC) using integrated digital error suppression (iDES). US 2014/065621 A1 describes methods for increasing fetal fraction in maternal blood.

### Summary

The present invention is set out in the appended claims. The technical information set out below may in some respects go beyond the scope of the present invention. The additional technical information is provided to place the present invention in a broader technical context and to illustrate possible related technical developments.

The present disclosure involves ctDNA assays that interrogate many genomic regions from a single sample with high precision and accuracy, while evaluating multiple forms of cancer-related genomic alterations including sequence mutations and structural alterations. The disclosure provides simplified yet robust methods that achieve high sensitivity and specificity by analyzing cancer genes using a limited pool of non-unique barcodes in combination with endogenous barcodes. Samples are captured and sequenced using high coverage next-generation sequencing to allow tumor-specific somatic mutations and translocations to be identified.

Analyses for sequence mutations or rearrangements can be performed together or separately, depending on the specific alterations of interest. The disclosed methods provide increase sensitivity and specificity of sequencing for diagnostic, forensic, genealogical, and clinical purposes.

The disclosed methods are particularly suited to accommodating low abundance sample DNA, such as in a liquid biopsy. Liquid biopsies assess DNA in the blood for circulating tumor DNA. Circulating tumor DNA (ctDNA) may enter the bloodstream through apoptosis of tumor cells and, when detected, allows diagnosis, genotyping, and disease monitoring without the need for traditional invasive biopsy procedures. However, ctDNA levels are generally quite low, particularly for early-stage tumors, which has made it difficult to rely on ctDNA for detection and analysis. The present invention, addresses that problem with methods for identifying rare mutations in samples containing limited amounts of DNA template. Methods of the invention reduce the effect of error rates that are inherent in massively parallel sequencing instruments. Without methods of the present disclosure, the error rates inherent in those instruments are generally too high to identify rare mutations in most samples.

Methods may include extracting and isolating cell-free DNA from a plasma sample and assigning an exogenous barcode to each fragment to generate a DNA library. The exogenous barcodes are from a limited pool of non-unique barcodes, for example 8 different barcodes. The barcoded fragments are differentiated based on the combination of their exogenous barcode and the endogenous barcode resulting from the genomic positions of fragment ends of each cell-free DNA molecule. The DNA library is redundantly sequenced and the sequences with matching barcodes are reconciled. The reconciled sequences are aligned to a human genome reference, and variants that exist in the aligned sequences are identified as bona fide mutations.

The invention recognizes that completely unique barcode sequences are unnecessary. Instead, a combination of predefined set of non-unique sequences together with the endogenous barcodes can provide the same level of sensitivity and specificity that unique barcodes could for biologically relevant DNA amounts. A limited pool of barcodes is more robust than a conventional unique set and easier to create and use. The methods may be used to assay a panel of well-characterized cancer genes, for example. The methods may also be used to evaluate sub-clonal mutations in tumor tissue.

Aspects of the invention involve a method for analyzing nucleic acids. The nucleic acid may be cell-free DNA, circulating tumor DNA, or RNA. The method involves obtaining a sample comprising nucleic acid fragments, introducing sets of non-unique barcodes to the fragments to generate a genomic library, identifying end portions of the fragments, sequencing the fragments to produce sequence reads, and aligning the sequence reads to identify a mutation.

The obtaining step may include obtaining a plasma sample, extracting nucleic acids, and fragmenting the nucleic acids. The introducing sets of non-unique barcodes step may include end repair, A-tailing, and adapter ligation. In some embodiments, the sets of non-unique barcodes consist of eight sets of non-unique barcodes. The barcodes may include sequencing adapters.

The step of identifying end portions may include hybrid capture or whole genome sequencing. The end portions of DNA fragments may include endogenous barcodes. Hybrid capture may involve a panel of well-characterized cancer genes including, for example, ABL1, AKT1, ALK, APC, AR, ATM, BCR, BRAF, CDH1, CDK4, CDK6, CDKN2A, CSF1R, CTNNB1, DNMT3A, EGFR, ERBB2, ERBB4, ESR1, EZH2, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MAP2K1, MET, MLH1, MPL, MYC, NPM1, NRAS, NTRK1, PDGFRA, PDGFRB, PIK3CA, PIK3R1, PTEN, PTPN11, RARA, RB1, RET, ROS1, SMAD4, SMARCB1, SMO, SRC, STK11, TERT, TP53, and/or VHL.

The sequencing step may involve single-end or paired-end sequencing. The sequencing step may involve redundant sequencing and using the redundant sequence reads to determine a consensus sequence. Redundant sequencing may be performed at a depth of 2x, 10x, 50x, 100x, or the like. The aligning step may include determining whether a locus of a barcoded fragment is identical across a predefined percentage of redundant sequence reads, such as 50%, 60%, 70%, 80%, 90%, 99%, or the like.

Also described herein but not part of the invention is a method for molecular barcoding, which includes the steps of obtaining a sample comprising nucleic acid fragments, providing a plurality of sets of non-unique barcodes, and tagging the nucleic acid fragments with the barcodes to generate a genomic library, wherein each nucleic acid fragment is tagged with the same barcode as another different nucleic acid fragment in the genomic library.

The plurality of sets is limited to twenty or fewer non-unique barcodes. In other embodiments, the plurality of sets is limited to ten or fewer non-unique barcodes.

The method may further include one or more of the following steps: identifying end portions of the fragments; redundantly sequencing the genomic library to produce a plurality of redundant sequence reads of each nucleic acid fragment; reconciling the redundant sequence reads of similarly-tagged nucleic acid fragments; and aligning the reconciled sequence reads to a reference to determine a consensus sequence.

### Brief Description of the Drawings

FIG. 1 shows a method of genotyping using non-unique barcodes in combination with endogenous barcodes.
FIG. 2 shows a method of barcoding according to the present disclosure.
FIGS. 3 and 4 show panels of well-characterized cancer genes for use with the invention.
FIG. 5 shows a flowchart of a method of genotyping.
FIG. 6 shows pan-cancer cell line sequence mutation observed and expected mutant allele frequency results.
FIG. 7 shows internal control breast cancer cell line observed and expected mutant allele frequency results.

### Detailed Description

High-throughput sequencing of circulating tumor DNA (ctDNA) promises to personalize cancer diagnosis and treatment, while eliminating the need for many invasive biopsy procedures. But low quantities of cell-free DNA (cfDNA) in the blood and the limitations of sequencing technology present challenges. The prevalence of sequencing artifacts limits the sensitivity of assays involving liquid biopsies of ctDNA. For example, Illumina sequencing has an error rate of up to 1%. Errors originate during template preparation, library preparation, and base-calling mistakes in sequencing. Those errors are particularly problematic when looking for low-frequency mutations. The methods disclosed herein address those and other problems.

Methods of the invention provide high-throughput profiling of a panel of cancer genes with high sensitivity and specificity of gene variants. The methods provide noninvasive genotyping and detection of ctDNA for both research and clinical purposes. The invention makes use of non-unique barcodes in conjunction with the target nucleic acids' endogenous barcodes to give high sensitivity and specificity in a genotyping assay. The methods are useful for low abundance sample DNA such as ctDNA.

The number of input molecules (i.e., genomic equivalents) of cfDNA is usually very small is plasma, making recovery of ctDNA a challenge. Library preparation and sequencing introduce errors that pose a significant obstacle for interrogating rare mutations. Methods of the invention achieve high detection limits in cfDNA (as low as 0.05-0.1%), and are able to find mutations in many malignancies that would go undetected with traditional methods. These methods improve the sensitivity and specificity of detecting low-frequency alleles. The invention recognizes that the combination of non-unique barcodes and molecular ends of DNA molecules can be used to distinguish DNA with a high level of sensitivity and specificity.

The methods generally involve tagging cfDNA fragments with a pool of non-unique barcodes and paired-end sequencing to identify the exogenous barcode and the fragment-specific endogenous barcode. While most prior barcoding methods are PCR based, the presently disclosed methods use a capture-based approach with a limited predefined set of barcodes layered on top of endogenous barcodes. Capture-based approaches involve generating a library of a genome and capturing certain regions. Such approaches are superior to PCR based strategies due to increased scalability, flexibility, and coverage uniformity. Capture-based methods can simultaneously interrogate thousands of genomic positions with high sensitivity and specificity. With this method, each end of a fragment is sequenced to distinguish the endogenous barcode sequence of the fragment ends, combined with the exogenous barcodes. Combining the pool of exogenous barcodes with the mapping positions of the DNA fragments provides all the complexity that is needed to identify fragments with sufficient sensitivity and specificity.

If for example there are 100 different endogenous barcodes on either end of the fragments-which can be generated by random shearing, exonuclease digestion, or natural fragmentation that may exist with cell free-DNA-then 10,000 different molecules could be evaluated using paired-end sequencing. Assigning a pool of 8 non-unique barcodes, for example, would thus yield 80,000 combinations. Such an assay can identify mutations in the 0.1 to 0.05% range. For assays that require that level of sensitivity, the present disclosure shows that a limited set of non-unique barcodes provides all the diversity that is needed in such an assay. According to the present invention, a small pool of non-unique exogenous barcodes can be layered onto endogenous end regions to provide a robust assay that achieves levels of sensitivity that are comparable to traditional, more complex barcoding schemes, while vastly reducing cost and complication. These numbers are merely an example and can be increased or decreased as necessary to suit a particular assay.

Sequencing may be performed at a depth of 2×, 10x, 50×, 100x, 1,000x, 10,000x, 50,000x or greater. Redundant sequence reads are compared and reconciled to distinguish somatic mutations from sequencing or other processing errors. If a mutation existed in the original DNA molecule, the mutation should be seen in every sequence read of that locus, notwithstanding any subsequent sequencing errors. A mutation can be called, for example, if a certain percentage of reads contain the putative mutation. The threshold percentage for making a mutation call can be 25%, 50%, 60%, 75%, 90%, 95%, 99%, and the like. The threshold can be set based on the number of sequence reads obtained and the particular needs of an assay. Likewise, mutations that do not occur in the template DNA would not be expected to appear in a significant percentage of reads, and those variants can be dismissed as sequencing errors, replication errors, or other processing errors. The consensus sequences can be determined by comparing and reconciling the sequence reads.

Methods of the invention involve isolating nucleic acids from a sample. Nucleic acids can be cfDNA that includes ctDNA. The methods are particularly useful for cfDNA, but other types of nucleic acids can be used as well, including RNA. Samples may include, for example, cell-free nucleic acid (including DNA or RNA) or nucleic acid isolated from a tumor tissue sample such as biopsied tissue, formalin fixed paraffin embedded tissue (FFPE), frozen tissue, cell lines, DNA and tumor grafts. Samples provided as FFPE blocks or frozen tissue may undergo pathological review to determine tumor cellularity. Tumors may be macro-dissected or micro-dissected to remove contaminating normal tissue. Samples may also be derived from patient lymphocytes, blood, saliva, cells obtained via buccal swab, or other unaffected tissue. Cell-free nucleic acids may be fragments of DNA or ribonucleic acid (RNA) which are present in the blood stream of a patient. In a preferred embodiment, the circulating cell-free nucleic acid is one or more fragments of DNA obtained from the plasma or serum of the patient.

The cell-free nucleic acid may be isolated according to techniques known in the art and include, for example, the QIAmp system from Qiagen (Venlo, Netherlands), the Triton/Heat/Phenol protocol (THP) (Xue, et al., Optimizing the Yield and Utility of Circulating Cell-Free DNA from Plasma and Serum", Clin. Chim. Acta., 2009; 404(2): 100-104), blunt-end ligation-mediated whole genome amplification (BL-WGA) (Li, et al., "Whole Genome Amplification of Plasma-Circulating DNA Enables Expanded Screening for Allelic Imbalance in Plasma", J. Mol Diagn. 2006 Feb; 8(1): 22-30), or the NucleoSpin system from Macherey-Nagel, GmbH & Co. KG (Duren, Germany). In an exemplary embodiment, a blood sample is obtained from the patient and the plasma is isolated by centrifugation. The circulating cell-free nucleic acid may then be isolated by any of the techniques above.

Generally, nucleic acid can be extracted, isolated, amplified, or analyzed by a variety of techniques such as those described by Green and Sambrook, Molecular Cloning: A Laboratory Manual (Fourth Edition), Cold Spring Harbor Laboratory Press, Woodbury, NY 2,028 pages (2012); or as described in U.S. Pat. 7,957,913; U.S. Pat. 7,776,616; U.S. Pat. 5,234,809; U.S. Pub. 2010/0285578; and U.S. Pub. 2002/0190663.

Nucleic acid obtained from biological samples may be fragmented to produce suitable fragments for analysis. Methods of fragmenting nucleic acids are known in the art. Template nucleic acids may be fragmented or sheared to desired length, using a variety of mechanical, chemical and/or enzymatic methods. Nucleic acid may be sheared by sonication, brief exposure to a DNase/RNase, hydroshear instrument, one or more restriction enzymes, transposase or nicking enzyme, exposure to heat plus magnesium, or by shearing. Nucleic acids may also be naturally fragmented as is the case for cell-free DNA. A biological sample may be lysed, homogenized, or fractionated in the presence of a detergent or surfactant as needed. Suitable detergents may include an ionic detergent (e.g., sodium dodecyl sulfate or N-lauroylsarcosine) or a nonionic detergent (such as the polysorbate 80 sold under the trademark TWEEN by Uniqema Americas (Paterson, NJ) or C₁₄H₂₂O(C₂H₄)ₙ, known as TRITON X-100). The resultant fragments may be any size, for example 10 bp, 50 bp, 100 bp, 500 bp, 1,000 bp, 5,000 bp, or greater. Shearing may be followed by end-repair and A-tailing. Sequencing adapters may be ligated according to standard sequencing protocols.

Hybrid capture probes using selectable oligonucleotides can be used to obtain nucleic acid of interest. See for example, Lapidus (U.S. patent number 7,666,593). Conventional methods for making and using hybridization probes can be found in standard laboratory manuals such as: Genome Analysis: A Laboratory Manual Series (Vols. 1-IV), Cold Spring Harbor Laboratory Press; PCR Primer: A Laboratory Manual, Cold Spring Harbor Laboratory Press; and Sambrook, J et al., (2001) Molecular Cloning: A Laboratory Manual, 2nd ed. (Vols. 1-3), Cold Spring Harbor Laboratory Press.

After processing steps such as those described above, nucleic acids can be sequenced. Sequencing may be by any method known in the art. DNA sequencing techniques include classic dideoxy sequencing reactions (Sanger method) using labeled terminators or primers and gel separation in slab or capillary, and next generation sequencing methods such as sequencing by synthesis using reversibly terminated labeled nucleotides, pyrosequencing, 454 sequencing, IlluminalSolexa sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Separated molecules may be sequenced by sequential or single extension reactions using polymerases or ligases as well as by single or sequential differential hybridizations with libraries of probes.

A sequencing technique that can be used includes, for example, use of sequencing-by-synthesis systems sold under the trademarks GS JUNIOR, GS FLX+ and 454 SEQUENCING by 454 Life Sciences, a Roche company (Branford, CT), and described by Margulies, M. et al., Genome sequencing in micro-fabricated high-density picotiter reactors, Nature, 437:376-380 (2005); U.S. Pat. 5,583,024; U.S. Pat. 5,674,713; and U.S. Pat. 5,700,673.

Other examples of DNA sequencing techniques include SOLiD technology by Applied Biosystems from Life Technologies Corporation (Carlsbad, CA) and ion semiconductor sequencing using, for example, a system sold under the trademark ION TORRENT by Ion Torrent by Life Technologies (South San Francisco, CA). Ion semiconductor sequencing is described, for example, in Rothberg, et al., An integrated semiconductor device enabling non-optical genome sequencing, Nature 475:348-352 (2011); U.S. Pub. 2010/0304982; U.S. Pub. 2010/0301398; U.S. Pub. 2010/0300895; U.S. Pub. 2010/0300559; and U.S. Pub. 2009/0026082.

Another example of a sequencing technology that can be used is Illumina sequencing. Illumina sequencing is based on the amplification of DNA on a solid surface using fold-back PCR and anchored primers. Adapters are added to the 5' and 3' ends of DNA that is either naturally or experimentally fragmented. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated. Sequencing according to this technology is described in U.S. Pat. 7,960,120; U.S. Pat. 7,835,871; U.S. Pat. 7,232,656; U.S. Pat. 7,598,035; U.S. Pat. 6,911,345; U.S. Pat. 6,833,246; U.S. Pat. 6,828,100; U.S. Pat. 6,306,597; U.S. Pat. 6,210,891; U.S. Pub. 2011/0009278; U.S. Pub. 2007/0114362; U.S. Pub. 2006/0292611; and U.S. Pub. 2006/0024681.

One limitation of sequencing technology is the prevalence of sequencing artifacts. A common approach to reducing sequencing artifacts is molecular barcoding. Most barcoding methods involve tagging DNA fragments with identifiers, which can be tracked throughout an assay, making it possible to distinguish somatic mutations from sequencing errors.

The term barcode encompasses both exogenous barcodes, which are introduced to sample DNA fragments, and endogenous barcodes, which are the end sequences that result from fragmenting DNA through biologic or experimental shearing. Barcodes may comprise any number of nucleotides, such as 2, 4, 8, 16, or more nucleotides.

Exogenous barcodes can be generated by methods known in the art. For example, they can be created by adding random nucleotides to a short sequence assembled on a substrate. They can be generated enzymatically by polymerase extension over a degenerate synthetic template or they can be synthesized in a single unit with adapter sequences. Synthesizing barcodes allows greater control over their composition, but can be expensive. Using a limited pool of barcodes thus allows an assay to be performed more cost-effectively.

Barcodes can be completely random or they can be engineered with certain predetermined sequences. They may have regions of randomness or semi-randomness and other fixed regions. The barcodes may include other regions, such as priming sites, adapters, or other complimentary regions that would facilitate further processing and analysis.

Exogenous barcodes may be attached to nucleic acid fragments by methods known in the art, such as via PCR or enzymatic ligation. They may be attached at one or both ends of the fragment. Barcode molecules may be commercially obtained, such as from Integrated DNA Technologies (Coralville, IA). In certain embodiments, one or more barcode is attached to each, any, or all of the fragments. A barcode sequence generally includes certain features that make the sequence useful in sequencing reactions. Methods of designing sets of barcode sequences are shown for example in U.S. Pat. 6,235,475. Attaching barcode sequences to nucleic acid templates is shown in U.S. Pub. 2008/0081330 and U.S. Pub. 2011/0301042. Methods for designing sets of barcode sequences and other methods for attaching barcode sequences are shown in U.S. Pats. 6,138,077; 6,352,828; 5,636,400; 6,172,214; 6235,475; 7,393,665; 7,544,473; 5,846,719; 5,695,934; 5,604,097; 6,150,516; RE39,793; 7,537,897; 6172,218; and 5,863,722. Barcodes for sequencing and copy number estimation are described in U.S. Pub. 2016/0046986.

The present disclosure makes use of non-unique barcodes to give high sensitivity and specificity in a genotyping assay. In other contexts, such as the publications referenced above, barcodes may be referred to as unique identifiers (UIDs). Here, we avoid that term because the exogenous barcodes of the present method do not have to be unique. Traditional barcoding methods emphasize the need to generate thousands or millions of barcode sequences or combinations to ensure with a high degree of certainty that no two fragments receive the same barcode. The present disclosure demonstrates that, contrary to conventional wisdom, smaller pools of non-unique barcodes layered onto endogenous barcodes can provide the same levels of diversity as traditional schemes, while reducing complexity and increasing assay robustness.

The present invention recognizes that while some level of barcoding is necessary to reduce background noise in a sequencing assay, prior art barcoding methods overestimate the problem. Traditionally methods involve generating several thousand or million barcode combinations. Generating those barcodes overcomplicates the genotyping assay and makes it less robust. The present disclosure shows that the same level of specificity can be achieved with significantly less complexity.

When the barcoded fragments are sequenced, a plurality of reads are generated. Reads may be between about 50 and 200 bases in length. In some embodiments, shorter reads can be obtained, for example, less than about 50 or about 30 bases in length. Some sequencing technologies can produce reads of several hundred or thousand bases in length.

A set of sequence reads can be analyzed by any suitable method known in the art. For example, in some embodiments, sequence reads are analyzed by hardware or software provided as part of a sequence instrument. In some embodiments, individual sequence reads are reviewed by sight (e.g., on a computer monitor).

Sequence assembly can be done by methods known in the art including reference-based assemblies, de novo assemblies, assembly by alignment, or combination methods. In some embodiments, sequence assembly uses the low coverage sequence assembly software (LOCAS) tool described by Klein, et al., in LOCAS-A low coverage sequence assembly tool for re-sequencing projects, PLoS One 6(8) article 23455 (2011), the contents of which are hereby incorporated by reference in their entirety. Sequence assembly is described in U.S. Pat.8,165,821; U.S. Pat. 7,809,509; U.S. Pat. 6,223,128; U.S. Pub. 2011/0257889; and U.S. Pub.2009/0318310.

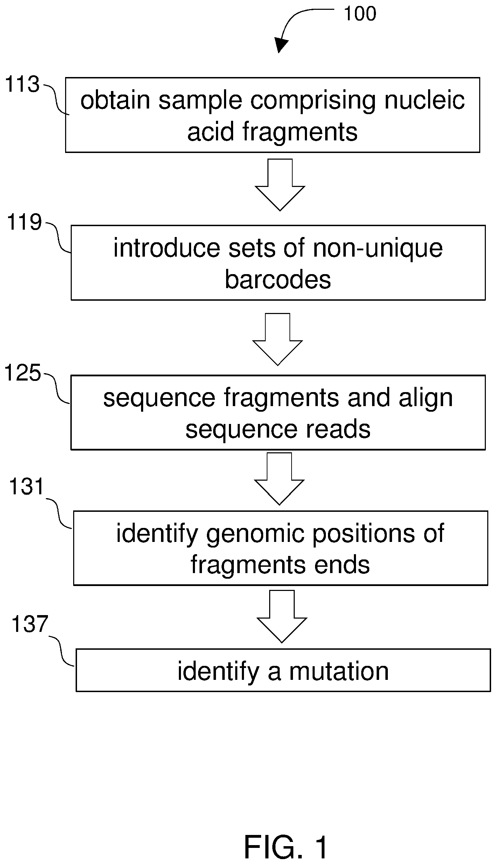

FIG. 1 shows a method 100 for analyzing nucleic acids in accordance with the present disclosure. The method 100 involves a step 113 of obtaining a sample that includes nucleic acid fragments. The step 113 may include obtaining a plasma sample from a patient and extracting nucleic acid fragments. The nucleic acids may include cell-free DNA, circulating tumor DNA, tumor DNA, or RNA. The fragments may be end-repaired, A-tailed, and ligated with an adapter. In step 119, sets of non-unique barcodes are introduced to generate a genomic library. In step 125, the fragments are sequenced to produce sequence reads and the sequence reads are aligned. Sequencing may involve redundantly sequencing each fragment. In step 131, genomic positions of fragment ends are identified. In step 137, a mutation that is present in multiple molecules is identified, as determined by a combination of non-unique barcodes and genomic position of fragment ends.

The method may include performing hybrid capture on the genomic library. Hybrid capture may involve a panel of well-characterized cancer genes, such as ABL1, AKT1, ALK, APC, AR, ATM, BCR, BRAF, CDH1, CDK4, CDK6, CDKN2A, CSF1R, CTNNB1, DNMT3A, EGFR, ERBB2, ERBB4, ESR1, EZH2, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MAP2K1, MET, MLH1, MPL, MYC, NPM1, NRAS, NTRK1, PDGFRA, PDGFRB, PIK3CA, PIK3R1, PTEN, PTPN11, RARA, RB1, RET, ROS1, SMAD4, SMARCB1, SMO, SRC, STK11, TERT, TP53, and VHL.

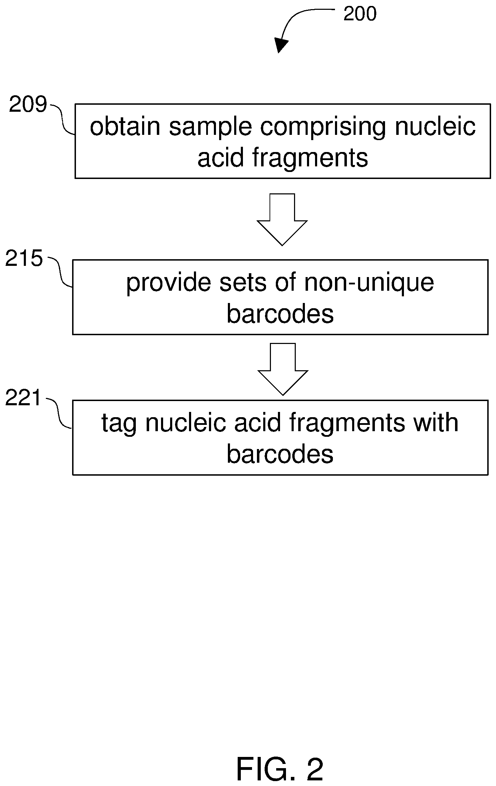

FIG. 2 shows a method 200 for molecular barcoding according to the present disclosure. The method 200 includes a step 209 of obtaining a sample having nucleic acid fragments and a step 215 of providing a plurality of sets of non-unique barcodes. In step 221, the nucleic acid fragments are tagged with the barcodes to generate a genomic library. Because there are a limited number of sets of non-unique barcodes (for example, eight different sets), each nucleic acid fragment gets tagged with the same barcode as at least one other different nucleic acid fragment in the genomic library. The exogenous barcodes are thus "non-unique." Genomic positions of the fragments can be identified by the endogenous barcodes that result from fragmentation of the nucleic acids.

In some embodiments, the method 200 further involves redundantly sequencing the genomic library to produce a plurality of redundant sequence reads of each nucleic acid fragment. The method 200 may further include reconciling the redundant sequence reads of similarly-tagged nucleic acid fragments. The method 200 may further include aligning the reconciled sequence reads to a reference to determine a consensus sequence.

The disclosed approach is useful for any sequencing assay where a high level of sensitivity and specificity is required. The methods are particularly useful for sequencing small amounts of cfDNA isolated from blood plasma and interrogating them for somatic mutations.

### Example

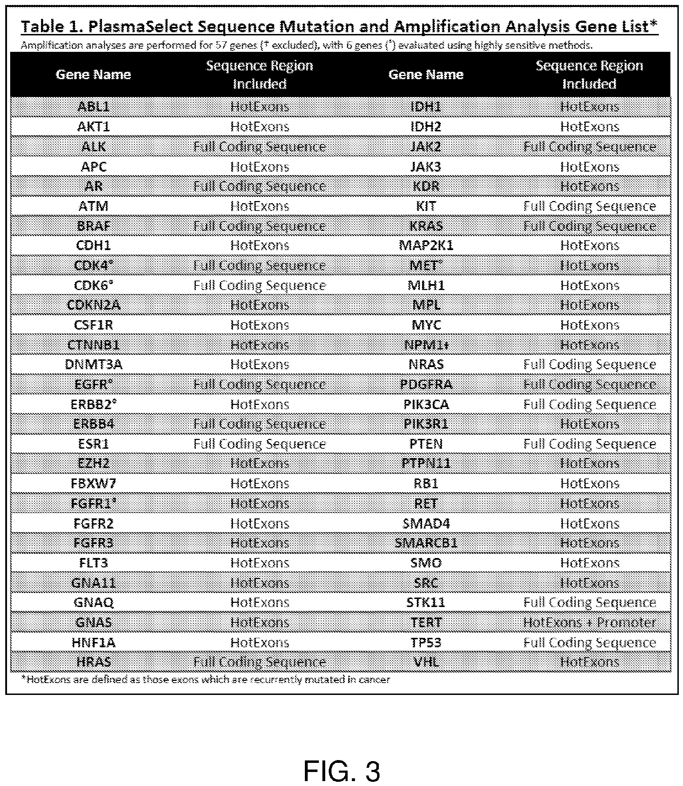

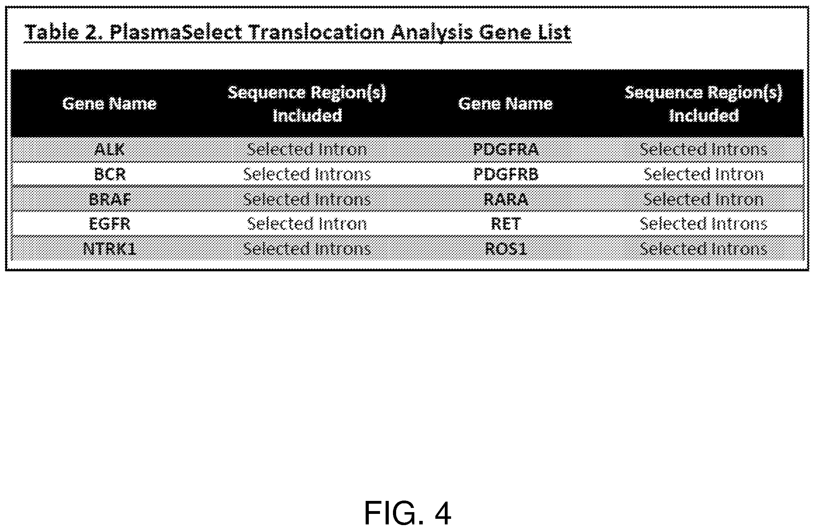

A validation study was conducted for research use. The goal of the study was to demonstrate that next-generation library preparation in combination with targeted gene capture using a panel is reproducible and accurate for sequencing on the Illumina HiSeq sequencing platform. The panel under study was a targeted panel of well-characterized cancer genes known as the PlasmaSelect^{™} panel, currently under development by PGDx (Baltimore, MD). Validation of this approach, using a combination cell-line derived and clinical plasma samples, enables the identification of tumor-specific sequence mutations, amplifications, and translocations in a set of genes relevant to clinical and biomedical cancer research. The scope of this method validation is to use this assay for research utilizing plasma samples derived from cancer patients for the evaluation of the genes indicated in FIGS. 3 and 4.

### Methods and Process Description

### 1. Sample Preparation, Library Generation and DNA Capture

### DNA Extraction and Processing

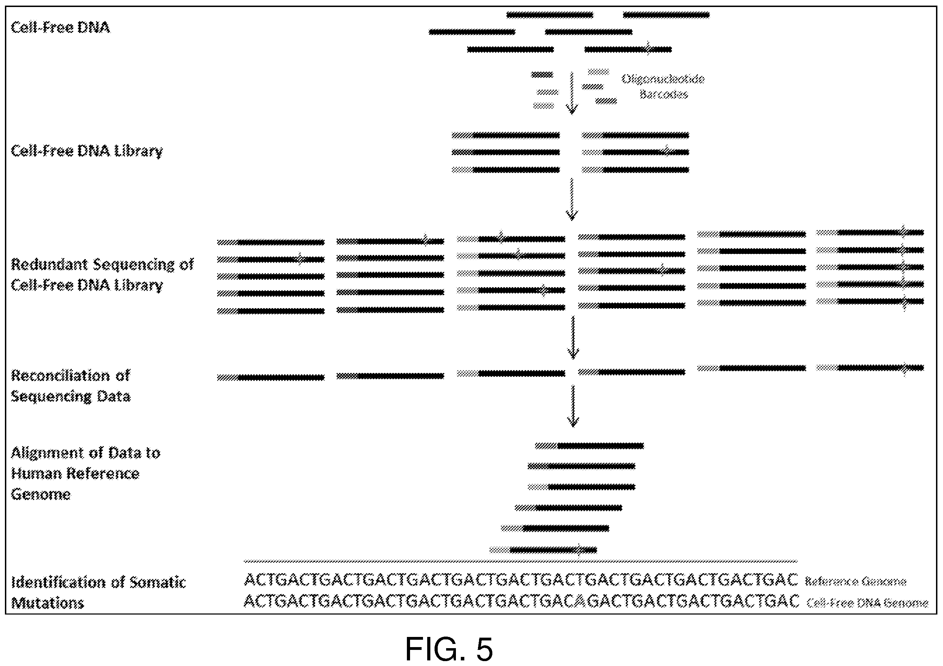

Targeted gene sequencing analyses of cell line derived and cell-free DNA (cfDNA) derived from plasma were performed to identify tumor-specific (somatic) alterations. Two technical challenges to implementing these approaches in the form of a liquid biopsy include the limited amount of DNA obtained and the low mutant allele frequency associated with these alterations. It has been documented that as few as several thousand genomic equivalents are obtained per milliliter of plasma, and the mutant allele frequency can range from <0.01% to >50% (Bettegowda et. al., 2014) total cfDNA. The disclosed techniques overcome this problem and improve test sensitivity, optimized methods for conversion of cell-free DNA into a genomic library, and digital sequencing approaches to improve the specificity of next-generation sequencing approaches. Utilizing digital sequencing technologies with redundant sequencing error-correction approaches effectively reduces the error rate introduced by next-generation sequencing, and allows for the accurate identification of sequence mutations (see FIG. 5, single-base and small insertions and deletions).

### Library Preparation and Targeted Capture

Briefly, cell-free DNA was extracted from cell line or plasma specimens and prepared into a genomic library suitable for next-generation sequencing with oligonucleotide barcodes through end-repair, A-tailing and adapter ligation. An in-solution hybrid capture, utilizing 120 base-pair (bp) RNA oligonucleotides was performed for both the sequence mutation panel (FIG. 3) and the structural alteration panel (FIG. 4).

### 2. Sequencing

Enriched cell line or plasma derived captured DNA libraries were sequenced using paired-end Illumina HiSeq2500 sequencing chemistry to an average target total coverage of either >20,000-fold for sequence mutations or >5,000-fold coverage for translocations, for each targeted base. Sequence data were mapped to the reference human genome sequence and coding and intronic regions were examined for somatic alterations.

### 3. Bioinformatics

The data was analyzed using sophisticated bioinformatics approaches, including novel genetic analysis methods, and proprietary data analysis algorithms, to sensitively and specifically identify tumor-specific alterations, and to integrate sequence information, genomic data, and cancer genes and pathways to provide the most complete and informative data set to guide patient management. Briefly, these steps involved:
1. Primary Processing of Next-Generation Sequencing Data
2. Alignment of Next-Generation Sequencing Data to the Human Reference Genome using ELAND and Novoalign
3. Analyses of Next-Generation Sequence Data for Sequence Mutations
4. Analyses of Next-Generation Sequence Data for Focal Amplifications
5. Analyses of Next-Generation Sequence Data for Translocations

### Study Plan and Sample Sets

### Sample Types

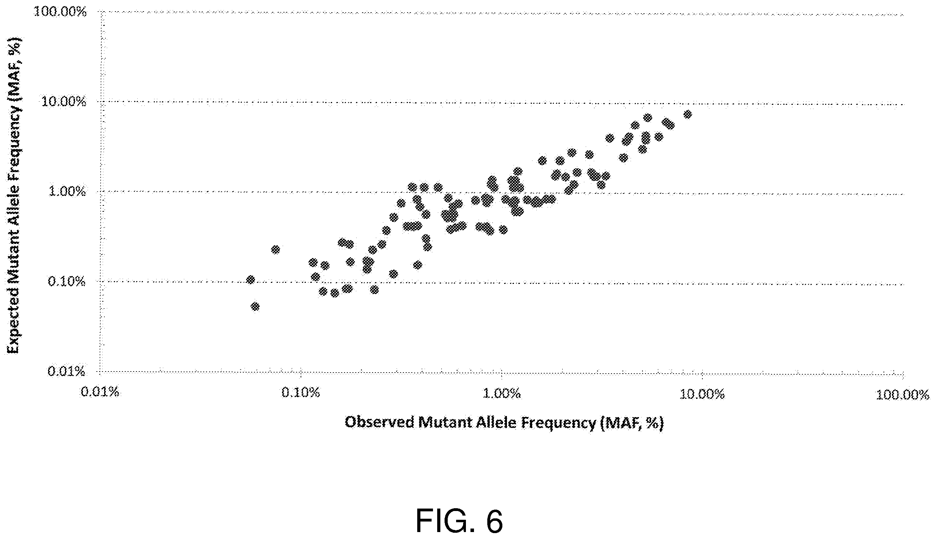

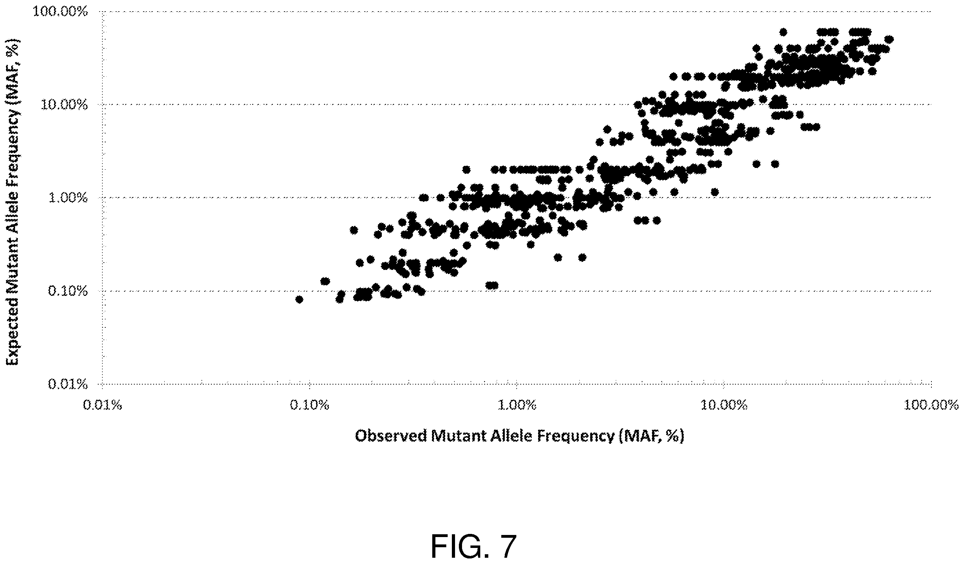

A validation study was performed using a combination of pan-cancer cell lines (Table 1), plasma derived from late-stage breast, colon, and lung cancer patients, as well as samples derived from healthy donors to evaluate assay performance (Tables 1-4 and FIGS. 6 and 7). Clinical samples, from both healthy donors and late-state cancer patients, were obtained retrospectively through ILSBio (Chestertown, MD). Cell line specimens were obtained from ATCC (Manassas, VA), from which DNA was extracted, sheared and purified to a fragment length profile consistent with cell-free DNA obtained from plasma. These samples were then evaluated using the PlasmaSelect^{™} R 64 panel in accordance with the associated Standard Operating Procedures (SOPs).

**Table 1. Pan-Cancer Cell Lines and Sequence Mutations.**

| **Tumor Type** | **Gene** | **Alteration** |
|---|---|---|
| Colorectal Adenocarcinoma | *KRAS* | p.Q61L |
| Colorectal Carcinoma | *KRAS* | p.A146T |
| Pancreatic Adenocarcinoma | *KRAS* | p.G12D |
| Melanoma | *NRAS* | p.G12V |
| Myeloma | *NRAS* | p.G13D |
| Small Cell Lung Carcinoma | *NRAS* | p.Q61R |
| Colorectal Adenocarcinoma | *EGFR* | p.G719S |
| Lung Adenocarcinoma | *EGFR* | p.ELR746del |
| Non-Small Cell Lung Adenocarcinoma | *EGFR* | p.T790M |
| Non-Small Cell Lung Adenocarcinoma | *EGFR* | p.L858R |
| Colorectal Adenocarcinoma | *BRAF* | p.V600E |
| Lung Adenocarcinoma | *ERBB2* | p.2327-2329InsTGT/p.G776V |

**Table 2. Sequence Mutation and Amplification Analyses Performed for the PlasmaSelect^{™} R64 Method Validation.**

| **Validation Component** | **Cell Type** | **Tumor Type** | **Experimental Tumor Purity** | **Total Input** |
|---|---|---|---|---|
| Specificity | Plasma | Normal | N/A | 9 |
| | Plasma | Normal | N/A | 10 |
| | Plasma | Normal | N/A | 10 |
| | Plasma | Normal | N/A | 8 |
| | Plasma | Normal | N/A | 10 |
| | Plasma | Normal | N/A | 9 |
| | Plasma | Normal | N/A | 9 |
| | Plasma | Normal | N/A | 9 |
| | Plasma | Normal | N/A | 9 |
| | Plasma | Normal | N/A | 8 |
| | Plasma | Normal | N/A | 9 |
| | Plasma | Normal | N/A | 10 |
| | Plasma | Normal | N/A | 10 |
| | Plasma | Normal | N/A | 10 |
| | Plasma | Normal | N/A | 10 |
| | Plasma | Normal | N/A | 11 |
| | Plasma | Normal | N/A | 10 |
| | Plasma | Normal | N/A | 10 |
| Accuracy | Cell Line Derived DNA | Breast | 100.0% | 250 ng |
| | Cell Line Derived DNA | Breast | 25.0% | 250 ng |
| | Cell Line Derived DNA | Breast | 20.0% | 250 |

| **Validation Component** | **Cell Type** | **Tumor Type** | **Experimental Tumor Purity** | **Total Input** |
|---|---|---|---|---|
| | Cell Line Derived DNA | Breast | 5.0% | 250 ng |
| | Cell Line Derived DNA | Breast | 2.0% | 250 ng |
| | Cell Line Derived DNA | Breast | 1.0% | 250 ng |
| | Multiple | Multiple | 100.0% | 250 ng |
| | Multiple | Multiple | 1.0% | 250 ng |
| Analytical Sensitivity | Cell Line Derived DNA | Breast | 2.0% | 250 ng |
| | Cell Line Derived DNA | Breast | 1.0% | 250 ng |
| | Cell Line Derived DNA | Breast | 0.5% | 250 ng |
| | Cell Line Derived DNA | Breast | 0.2% | 250 ng |
| | Cell Line Derived DNA | Breast | 0.1% | 250 ng |
| | Cell Line Derived DNA | Breast | 2.0% | 250 ng |
| | Cell Line Derived DNA | Breast | 1.0% | 250 ng |
| | Cell Line Derived DNA | Breast | 0.5% | 250 ng |
| | Cell Line Derived DNA | Breast | 0.2% | 250 ng |
| | Cell Line Derived DNA | Breast | 0.1% | 250 ng |
| | Cell Line Derived DNA | Breast | 10.0% | 25 ng |
| | Cell Line Derived DNA | Breast | 5.0% | 25 ng |
| | Cell Line Derived DNA | Breast | 2.0% | 25 ng |
| | Cell Line Derived DNA | Breast | 1.0% | 25 ng |
| | Cell Line Derived DNA | Breast | 0.5% | 25 ng |
| | Cell Line Derived DNA | Breast | 10.0% | 25 ng |
| | Cell Line Derived DNA | Breast | 5.0% | 25 ng |
| | Cell Line Derived DNA | Breast | 2.0% | 25 ng |
| | Cell Line Derived DNA | Breast | 1.0% | 25 ng |
| | Cell Line Derived DNA | Breast | 0.5% | 25 ng |
| | Cell Line Derived DNA | Breast | 60% | 250 ng |
| | Cell Line Derived DNA | Breast | 40% | 250 ng |
| | Cell Line Derived DNA | Breast | 20% | 250 ng |
| | Cell Line Derived DNA | Breast | 60% | 250 ng |
| | Cell Line Derived DNA | Breast | 40% | 250 ng |
| | Cell Line Derived DNA | Breast | 20% | 250 ng |
| | Cell Line Derived DNA | Breast | 60% | 25 ng |
| | Cell Line Derived DNA | Breast | 40% | 25 ng |
| | Cell Line Derived DNA | Breast | 20% | 25 ng |
| | Cell Line Derived DNA | Breast | 60% | 25 ng |
| | Cell Line Derived DNA | Breast | 40% | 25 ng |
| | Cell Line Derived DNA | Breast | 20% | 25 ng |
| | Multiple | Multiple | 1.0% | 250 ng |
| | Multiple | Multiple | 0.5% | 250 ng |
| | Multiple | Multiple | 0.2% | 250 ng |
| | Multiple | Multiple | 0.1% | 250 ng |
| | Multiple | Multiple | 5.0% | 25 ng |
| | Multiple | Multiple | 2.0% | 25 ng |
| | Multiple | Multiple | 1.0% | 25 ng |
| | Multiple | Multiple | 0.5% | 25 ng |
| Precision and Robustness | Cell Line Derived DNA | Breast | 2.0% | 150 ng |
| | Cell Line Derived DNA | Breast | 20.0% | 100 ng |
| | Cell Line Derived DNA | Breast | 2.0% | 150 ng |
| | Cell Line Derived DNA | Breast | 20.0% | 100 ng |
| | Cell Line Derived DNA | Breast | 2.0% | 150 ng |
| | Cell Line Derived DNA | Breast | 20.0% | 100 ng |

| | | | | |
|---|---|---|---|---|
| *Tumor purity for cell line samples was generated by titrating the tumor and normal DNA in the indicated ratio for a given DNA input to result in the indicated tumor purity. Manufacturer guidelines were followed for reagents used in library preparation. | | | | |

**Table 3. Rearrangement Analyses Performed for the PlasmaSelect^{™} R 64 Method Validation.**

| **Validation Component** | **Sample Type** | **Tumor Type** | **Experimental Tumor Purity** | **Total Input (ng, mL)** |
|---|---|---|---|---|
| Specificity | Plasma | Normal | N/A | 9 |
| | Plasma | Normal | N/A | 10 |
| | Plasma | Normal | N/A | 10 |
| | Plasma | Normal | N/A | 8 |
| | Plasma | Normal | N/A | 10 |
| | Plasma | Normal | N/A | 9 |
| | Plasma | Normal | N/A | 9 |
| | Plasma | Normal | N/A | 9 |
| | Plasma | Normal | N/A | 9 |
| | Plasma | Normal | N/A | 8 |
| | Plasma | Normal | N/A | 9 |
| | Plasma | Normal | N/A | 10 |
| | Plasma | Normal | N/A | 10 |
| | Plasma | Normal | N/A | 10 |
| | Plasma | Normal | N/A | 10 |
| | Plasma | Normal | N/A | 11 |
| | Plasma | Normal | N/A | 10 |
| | Plasma | Normal | N/A | 10 |
| Accuracy | Cell Line Derived DNA | CML | 100.0% | 250 ng |
| | Cell Line Derived DNA | CML | 2.0% | 250 ng |
| | Cell Line Derived DNA | CML | 1.0% | 250 ng |
| | Cell Line Derived DNA | CML | 100.0% | 250 ng |
| | Cell Line Derived DNA | CML | 2.0% | 250 ng |
| | Cell Line Derived DNA | CML | 1.0% | 250 ng |
| | Cell Line Derived DNA | NSCLC | 20.0% | 250 ng |
| | Cell Line Derived DNA | NSCLC | 1.0% | 250 ng |
| Analytical Sensitivity | Cell Line Derived DNA | CML | 1.0% | 250 ng |
| | Cell Line Derived DNA | CML | 0.5% | 250 ng |
| | Cell Line Derived DNA | CML | 0.1% | 250 ng |
| | Cell Line Derived DNA | CML | 1.0% | 250 ng |
| | Cell Line Derived DNA | CML | 0.5% | 250 ng |
| | Cell Line Derived DNA | CML | 0.1% | 250 ng |
| | Cell Line Derived DNA | NSCLC | 1.0% | 250 ng |
| | Cell Line Derived DNA | NSCLC | 0.5% | 250 ng |
| | Cell Line Derived DNA | NSCLC | 0.1% | 250 ng |
| | Cell Line Derived DNA | CML | 2.0% | 25 ng |
| | Cell Line Derived DNA | CML | 1.0% | 25 ng |
| | Cell Line Derived DNA | CML | 0.5% | 25 ng |
| | Cell Line Derived DNA | CML | 2.0% | 25 ng |
| | Cell Line Derived DNA | CML | 1.0% | 25 ng |
| | Cell Line Derived DNA | CML | 0.5% | 25 ng |
| | Cell Line Derived DNA | NSCLC | 2.0% | 25 ng |
| | Cell Line Derived DNA | NSCLC | 1.0% | 25 ng |
| | Cell Line Derived DNA | NSCLC | 0.5% | 25 ng |
| Precision and Robustness | Cell Line Derived DNA | CML | 2.0% | 150 ng |
| | Cell Line Derived DNA | CML | 5.0% | 25 ng |
| | Cell Line Derived DNA | CML | 2.0% | 150 ng |
| | Cell Line Derived DNA | CML | 5.0% | 25 ng |
| | Cell Line Derived DNA | CML | 2.0% | 150 ng |
| | Cell Line Derived DNA | CML | 5.0% | 25 ng |

| | | | | |
|---|---|---|---|---|
| Tumor purity for cell line samples was generated by titrating the tumor and normal DNA in the indicated ratio for a given DNA input to result in the indicated tumor purity. Manufacturer guidelines were followed for reagents used in library preparation. | | | | |

**Table 4. Clinical Plasma Samples Obtained from 18 Breast, Colon and Lung Cancer Patients.**

| **Speciment Type** | **Clinical Diagnosis** | **Clinical Stage** | **Total Plasma (mL)** |
|---|---|---|---|
| Blood | Breast Cancer | IIIA | 6 |
| Blood | Breast Cancer | IIIA | 12 |
| Blood | Breast Cancer | IIIA | 12 |
| Blood | Breast Cancer | IIIC | 7 |
| Blood | Lung Cancer | IIIA | 8 |
| Blood | Colon Cancer | IIIB | 6 |
| Blood | Colon Cancer | IIIB | 6 |
| Blood | Colon Cancer | IIIB | 6 |
| Blood | Colon Cancer | IV | 12 |
| Blood | Colon Cancer | IIIA | 12 |
| Blood | Colon Cancer | IIIA | 5 |
| Blood | Colon Cancer | IIIA | 5 |
| Blood | Colon Cancer | IV | 10 |
| Blood | Colon Cancer | IIIB | 5 |
| Blood | Colon Cancer | IIIA | 7 |
| Blood | Colon Cancer | IIIB | 7 |
| Blood | Colon Cancer | IIIB | 9 |
| Blood | Colon Cancer | IIIB | 7 |

### Test Performance Acceptance Criteria:

### 1. Accuracy:

### Sequence Mutations

Accuracy was assessed by comparing the results from a proprietary cell line between the targeted capture panel and next-generation sequencing method and published, independently obtained Sanger sequencing results for this case. A total of 19 positions known to be mutated in the proprietary cell line are included in the targeted panel, and were evaluated at 1%, 2%, 5%, 20%, 25%, and 100% tumor purity using 250ng of DNA. Furthermore, the combined cancer cell line containing 12 sequence mutations was evaluated at 100% and 1% tumor purity using 250ng of DNA. Finally, specificity was evaluated through analysis of 18 plasma samples derived from healthy donors, none of which would be expected to harbor any somatic alterations.

### Performance Metrics

| | |
|---|---|
| Sensitivity | 100.0% |
| Specificity (Contrived Cases) | 99.9997% |
| Specificity (Healthy Donors) | 99.9996% |

### Amplifications

Accuracy was assessed by comparing the results from the proprietary cell line between the targeted capture panel and next-generation sequencing method and published, independently obtained SNP array results for this case. There were 3 amplifications included in the targeted regions of interest, and were evaluated at 20%, 25%, and 100% tumor purity using 250ng of DNA. Additionally, specificity was evaluated through analysis of 18 plasma sample derived from healthy donors, none of which would be expected to harbor any somatic alterations.

### Performance Metrics

| | |
|---|---|
| Sensitivity | 100.0% |
| Specificity (Contrived Cases) | 91.7% |
| Specificity (Healthy Donors) | 100.0% |

### Rearrangements

Accuracy was assessed by comparing the results from various proprietary cell lines between the targeted capture panel and next-generation sequencing method and published, independently obtained results for these cases (Shibata et. al., 2010 and Koivunen et. al., 2008) at a combination of 1%, 2%, 20%, and 100% tumor purity using 250ng of DNA. Additionally, specificity was evaluated through analysis of 18 plasma sample derived from healthy donors, none of which would be expected to harbor any somatic alterations.

### Performance Metrics

| | |
|---|---|
| Sensitivity | 100.0% |
| Specificity (Contrived Cases) | 100.0% |
| Specificity (Healthy Donors) | 99.7% |

### 2. Analytical Sensitivity (Limit of Detection):

### Sequence Mutations

Analytical sensitivity was assessed by comparing the results from the proprietary cell line between the targeted capture panel and next-generation sequencing method and published, independently obtained Sanger sequencing results for this case. A total of 19 positions known to be mutated in the proprietary cell line are included in the targeted panel, and were evaluated at 0.1%, 0.2%, 0.5%, 1%, and 2% tumor purity in duplicate using 250ng of DNA as well as 0.5%, 1%, 2%, 5%, and 10% tumor purity in duplicate using 25ng of DNA. Furthermore, the combined mutant cell line containing 12 sequence mutations was evaluated at 0.1%, 0.2%, 0.5%, and 1% tumor purity using 250ng of DNA and 0.5%, 1.0%, 2.0%, and 5% tumor purity using 25ng of DNA.

### Performance Metric

| | |
|---|---|
| Analytical Sensitivity | 99.4% |

### Amplifications

Analytical sensitivity was assessed by comparing the results from the proprietary cell line between the targeted capture panel and next-generation sequencing method and published, independently obtained SNP array results for this case. There are 3 amplifications included in the targeted regions of interest, and were evaluated at 60%, 40% and 20% tumor purity in duplicate using 250ng of DNA as well as 60%, 40% and 20% tumor purity in duplicate using 25ng of DNA.

### Performance Metric

| | |
|---|---|
| Analytical Sensitivity | 97.2% |

### Rearrangements

Analytical sensitivity was assessed by comparing the results from various proprietary cell lines between the targeted capture panel and next-generation sequencing method and published, independently obtained results for these cases (Shibata et. al., 2010 and Koivunen et. al., 2008) at a combination of 0.1%, 0.5%, and 1.0% tumor purity using 250ng of DNA and 0.5%, 1.0%, and 2.0% tumor purity using 25ng of DNA.

### Performance Metric

| | |
|---|---|
| Analytical Sensitivity | 94.4% |

### 3. Precision and Robustness (Intra-Assay and Inter-Assay Reproducibility):

### Sequence Mutations

Precision and robustness were assessed by comparing the results from the proprietary cell line between the targeted capture panel and next-generation sequencing method and published, independently obtained Sanger sequencing results for this case. A total of 19 positions known to be mutated in the proprietary cell line were included in the targeted panel, and were evaluated at 2% tumor purity using 150ng of DNA both within and across sample preparations (different operator on different days).

### Performance Metrics

| | |
|---|---|
| Intra-Assay Concordance | 100.0% |
| Inter-Assay Concordance | 100.0% |

### Amplifications

Precision and robustness was assessed by comparing the results from the proprietary cell line between the targeted capture panel and next-generation sequencing method and published, independently obtained SNP array results for this case. There are 3 amplifications included in the targeted regions of interest, and were evaluated at 20% tumor purity using 100ng of DNA both within and across sample preparations (different operator on different days).

### Performance Metrics

| | |
|---|---|
| Intra-Assay Concordance | 94.7% |
| Inter-Assay Concordance | 89.5% |

### Rearrangements

Precision and robustness were assessed by comparing the results from various proprietary cell lines between the targeted capture panel and next-generation sequencing method and published, independently obtained results for these cases (Shibata et. al., 2010 and Koivunen et. al., 2008) at 2% and 5% tumor purity using 25ng and 150ng of DNA both within and across sample preparations (different operator on different days).

### Performance Metrics

| | |
|---|---|
| Intra-Assay Concordance | 100.0% |
| Inter-Assay Concordance | 100.0% |

### 4. Failure Rate

In total, there were 113 sequence panel (PS_Seq2) and 112 structural panel (PS_Str2) next-generation sequencing libraries generated with 6 library and processing failures (6/225, 2.7%).

### 5. Comparison of Blood Collection Tube Type

In order to evaluate the impact of blood collection tube type on the performance of the PlasmaSelect^{™} R 64 approach, 4x10 ml blood draws were obtained from 9 cancer patients, with 2x10 ml blood collected in K₂EDTA blood collection tubes, and 2x10 ml collected in Streck blood collection tubes and processed into plasma according to PGDx (K₂EDTA) or the manufacturer's specifications (Streck). These data demonstrated very high concordance between the overall reported results.

### Performance Metrics

| | |
|---|---|
| Sequence Mutation Concordance | 100.0% [MAF ≥0.50%] |
| Amplification Concordance | 98.8% |
| Rearrangement Concordance | 100.0% |

### 6. Stability:

Manufacture guidelines were followed for reagents used in sample library preparation and all samples were collected following the same sample protocol and handling procedures.

FIG. 6 shows pan-cancer cell line sequence mutation observed and expected mutant allele frequency results. The calculated mutant allele frequency (MAF) was compared to the expected MAF for the cases evaluated in the accuracy, analytical sensitivity, and precision and robustness method validation studies from the combined cancer cell lines (n=12 expected alterations for each case).

FIG. 7 shows internal control breast cancer cell line observed and expected mutant allele frequency results. The calculated mutant allele frequency (MAF) was compared to the expected MAF for the cases evaluated in the accuracy, analytical sensitivity, and precision and robustness method validation studies from the combine cancer cell line (n=19 expected alterations for each case).

### Conclusions and Recommendations

The PlasmaSelect^{™} assay has been validated to achieve high levels of sensitivity and specificity for detection of sequence mutations (SBS/indels), amplifications, and translocations in the cell-free DNA obtained from the plasma of cancer patients for liquid biopsy analyses.

### Performance Metrics (minimum sample input of 25ng):

**Table 5. Summary of PlasmaSelect^{™} R 64 Performance Metrics**

| **Performance Specification** | **Mutant Allele Fraction** | **Sensitivity** | **Specificity** |
|---|---|---|---|
| Sequence Mutations (SBS/Indel) | ≥0.50% | 99.4% | >99.999%* |
| Rearrangements | ≥0.50% | 94.4% | >99% |
| Amplifications (≥4-fold) | ≥20% | 97.2% | >99% |
| Amplifications (≥4-fold) | <20% | *varies depending on level of amplification and tumor content* | |

| | | | |
|---|---|---|---|
| *Per-base specificity provided for sequence mutation analyses [99,359 bases evaluated] | | | |

### Equivalents

The foregoing embodiments are therefore to be considered in all respects illustrative rather than limiting on the invention described herein.

## Claims

1. A method of analyzing nucleic acids in a sample of nucleic acid fragments, the method comprising:
introducing a limited pool of non-unique exogenous barcodes to the nucleic acid fragments wherein the limited pool comprises twenty or fewer sets of non-unique exogenous barcodes;
generating a genomic library;
identifying end portions of the nucleic acid fragments; sequencing the nucleic acid fragments to produce sequence reads; and
aligning the sequence reads to identify a variant,
wherein the end portions of the DNA fragments include endogenous barcodes.

2. The method of claim 1, wherein the variant identified is selected from tumour-specific somatic mutation, amplification, and translocation.

3. The method of claim 1, wherein identifying genomic positions of the fragments comprises hybrid capture or whole genome sequencing.

4. The method of claim 3, wherein hybrid capture involves a panel of well-characterized cancer genes.

5. The method of claim 4, wherein the cancer genes include ABL1, AKT1, ALK, APC, AR, ATM, BCR, BRAF, CDH1, CDK4, CDK6, CDKN2A, CSF1R, CTNNB1, DNMT3A, EGFR, ERBB2, ERBB4, ESR1, EZH2, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MAP2K1, MET, MLH1, MPL, MYC, NPM1, NRAS, NTRK1, PDGFRA, PDGFRB, PIK3CA, PIK3R1, PTEN, PTPN11, RARA, RB1, RET, ROS1, SMAD4, SMARCB1, SMO, SRC, STK11, TERT, TP53, and VHL.

6. The method of claim 1, wherein sequencing comprises single-end or paired-end sequencing.

7. The method of claim 1, wherein nucleic acid comprises cell-free DNA, circulating tumor DNA, tumor-derived DNA, or RNA.

8. The method of claim 1, wherein the limited pool comprises eight sets of non-unique exogenous barcodes.

## Patentansprüche

1. Verfahren zur Analyse von Nukleinsäuren in einer Probe von Nukleinsäurefragmenten, wobei das Verfahren Folgendes umfasst:
Einführen eines begrenzten Pools nicht einmaliger exogener Strichcodes in die Nukleinsäurefragmente, wobei der begrenzte Pool zwanzig oder weniger Sätze nicht einmaliger exogener Strichcodes umfasst;
Erzeugen einer genomischen Bibliothek;
Identifizieren von Endteilen der Nukleinsäurefragmente;
Sequenzieren der Nukleinsäurefragmente unter Erhalt von Sequenz-Reads; und
Ausrichten der Sequenz-Reads zur Identifizierung einer Variante,
wobei die Endteile der DNA-Fragmente endogene Strichcodes enthalten.

2. Verfahren nach Anspruch 1, wobei die identifizierte Variante aus tumorspezifischer somatischer Mutation, Amplifikation und Translokation ausgewählt ist.

3. Verfahren nach Anspruch 1, wobei Identifizieren genomischer Positionen der Fragmente Hybridfang oder Sequenzierung ganzer Genome umfasst.

4. Verfahren nach Anspruch 3, wobei Hybridfang unter Beteiligung einer Palette gut charakterisierter Krebsgene erfolgt.

5. Verfahren nach Anspruch 4, wobei zu den Krebsgenen ABL1, AKT1, ALK, APC, AR, ATM, BCR, BRAF, CDH1, CDK4, CDK6, CDKN2A, CSF1R, CTNNB1, DNMT3A, EGFR, ERBB2, ERBB4, ESR1, EZH2, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MAP2K1, MET, MLH1, MPL, MYC, NPM1, NRB, NTRK1, PDGFRA, PDGFRB, PIK3CA, PIK3R1, PTEN, PTPN11, RARA, RB1, RET, ROS1, SMAD4, SMARCB1, SMO, SRC, STK11, TERT, TP53 und VHL gehören.

6. Verfahren nach Anspruch 1, wobei Sequenzieren Single-end- oder Paired-end-Sequenzieren umfasst.

7. Verfahren nach Anspruch 1, wobei Nukleinsäure zellfreie DNA, zirkulierende Tumor-DNA, aus Tumoren gewonnene DNA oder RNA umfasst.

8. Verfahren nach Anspruch 1, wobei der begrenzte Pool acht Sätze nicht einmaliger exogener Strichcodes umfasst.

## Revendications

1. Procédé d'analyse d'acides nucléiques dans un échantillon de fragments d'acides nucléiques, le procédé comprenant :
l'introduction d'un groupe limité de codes-barres exogènes non uniques aux fragments d'acides nucléiques, le groupe limité comprenant vingt ensembles ou moins de codes-barres exogènes non uniques ;
la génération d'une bibliothèque génomique ;
l'identification de parties terminales des fragments d'acides nucléiques ; le séquençage des fragments d'acides nucléiques pour produire des lectures de séquence ; et
l'alignement des lectures de séquence pour identifier un variant,
les parties terminales des fragments d'ADN comprenant des codes-barres endogènes.

2. Procédé selon la revendication 1, dans lequel le variant identifié est choisi parmi une mutation somatique spécifique à une tumeur, une amplification et une translocation.

3. Procédé selon la revendication 1, dans lequel l'identification de positions génomiques des fragments comprend une capture hybride ou un séquençage du génome entier.

4. Procédé selon la revendication 3, dans lequel la capture hybride implique un panel de gènes cancéreux bien caractérisés.

5. Procédé selon la revendication 4, dans lequel les gènes cancéreux comprennent ABL1, AKT1, ALK, APC, AR, ATM, BCR, BRAF, CDH1, CDK4, CDK6, CDKN2A, CSF1R, CTNNB1, DNMT3A, EGFR, ERBB2, ERBB4, ESR1, EZH2, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MAP2K1, MET, MLH1, MPL, MYC, NPM1, NRAS, NTRK1, PDGFRA, PDGFRB, PIK3CA, PIK3R1, PTEN, PTPN11, RARA, RB1, RET, ROS1, SMAD4, SMARCB1, SMO, SRC, STK11, TERT, TP53, et VHL.

6. Procédé selon la revendication 1, dans lequel le séquençage comprend un séquençage à extrémité unique ou à extrémités appariées.

7. Procédé selon la revendication 1, dans lequel l'acide nucléique comprend un ADN acellulaire, un ADN tumoral circulant, un ADN dérivé d'une tumeur ou un ARN.

8. Procédé selon la revendication 1, dans lequel le groupe limité comprend huit ensembles de codes-barres exogènes non uniques.
